# EUROPÄISCHE PATENTANMELDUNG

(11) **EP 2 335 555 A1**
(43) Veröffentlichungstag der Anmeldung: **22.06.2011**
(21) Anmeldenummer: 10194649.9
(22) Anmeldetag: 13.12.2010
(51) Int. Cl.: A61B 1/00, A61B 5/00, A61B 5/1495, G01N 21/64

(54) **Verfahren zum Prüfen einer optischen Vorrichtung**

(30) Priorität: 16.12.2009 DE 102009058663
(71) Anmelder: Karl Storz GmbH & Co. KG, 78532 Tuttlingen (DE)
(72) Erfinder: Beck, Gerd, 78532, Tuttlingen (DE); Ehrhardt, André, 78532, Tuttlingen (DE); Glöggler, Bernhard, 78532, Tuttlingen (DE); Martin, Uwe, 78532, Tuttlingen (DE)

(57) **Zusammenfassung**

Bei einem Verfahren zum Prüfen eines optischen Untersuchungssystems mit einer Lichtquelle (22) und einer bildgebenden Einrichtung (10) zur optischen Untersuchung eines Objekts in remittiertem Licht und Fluoreszenzlicht, wobei die Lichtquelle (22) ausgebildet ist, um Beleuchtungslicht mit einem ersten vorbestimmten Beleuchtungsspektrum (83L) oder mit einem zweiten vorbestimmten Beleuchtungsspektrum (83F) zu erzeugen, und wobei ein Beobachtungs-Strahlengang der bildgebende Einrichtung (10) ein erstes vorbestimmtes Transmissionsspektrum (84L) oder ein zweites vorbestimmtes Transmissionsspektrum (84F) aufweist, wird die bildgebende Einrichtung gegenüber einer Referenzoberfläche (72) mit einem Indikatorbereich (75) mit einer wellenlängenabhängigen optischen Eigenschaft angeordnet, wobei die optische Eigenschaft sich zwischen einem ersten Schwerpunkt eines ersten Produkts (87) aus dem ersten vorbestimmten Beleuchtungsspektrum (83L) und dem ersten vorbestimmten Transmissionsspektrum (84L) und einem zweiten Schwerpunkt eines zweiten Produkts (88) aus dem zweiten vorbestimmten Beleuchtungsspektrums (83F) und dem zweiten vorbestimmten Transmissionsspektrum (84F) wesentlich ändert. Die Referenzoberfläche (72) wird mit Beleuchtungslicht der Lichtquelle (22) beleuchtet. Ein Bild der Referenzoberfläche (72) wird mittels der bildgebenden Einrichtung (10) erfasst. Anhand des erfassten Bilds wird bestimmt, welches Beleuchtungsspektrum und welches Transmissionsspektrum im Beobachtungs-Strahlengang beim Erfassen des Bilds vorlag,

## Beschreibung

Die vorliegende Erfindung bezieht sich auf ein Verfahren zum Prüfen eines optischen Untersuchungssystems mit einer Lichtquelle und einer bildgebenden Einrichtung zur optischen Untersuchung eines Objekts innerhalb und außerhalb der Medizin. Die vorliegende Erfindung bezieht sich insbesondere auf ein Verfahren zum Prüfen eines Endoskopiesystems mit einer Lichtquelle und einem Endoskop.

Zur Endoskopie in medizinischen oder nicht-medizinischen Anwendungen - im zweiten Fall auch als Boroskopie bezeichnet - werden Endoskopiesysteme aus jeweils einem Endoskop und einer Lichtquelle verwendet. Die Lichtquelle kann in das Endoskop, insbesondere in dessen distales Ende, integriert sein oder als separate Einheit bereitstehen, die über ein Lichtleitkabel mit dem Endoskop optisch gekoppelt ist. Licht der Lichtquelle tritt am distalen Ende des Endoskops aus und beleuchtet dort ein zu betrachtendes Objekt. Vom Objekt remittiertes Licht wird von einer Optik am distalen Ende des Endoskops aufgefangen und auf einen lichtempfindlichen Bildsensor geleitet oder, beispielsweise mittels eines geordneten Bündels von Lichtwellenleitern oder einer Stablinsenoptik, zum proximalen Ende des Endoskops übertragen. Im zweiten Fall ist das vom Objekt remittierte Licht am proximalen Ende des Endoskops über ein Okular beobachtbar oder wird mittels einer Kamera erfasst. Alternativ oder zusätzlich zu remittiertem Licht kann auch vom Objekt emittiertes Licht beobachtet werden, insbesondere Fluoreszenzlicht.

Die Qualität eines mittels eines Endoskopiesystems erfassten Bilds, insbesondere Helligkeit, Helligkeits- und Farbkontrast, Signal-Rausch-Abstand, Farbtreue und Auflösung bzw. Schärfe, hängt vom betrachteten Objekt, insbesondere seinen optischen Eigenschaften, und vor allem vom Endoskopiesystem ab. Relevante Faktoren sind beispielsweise die Funktionsfähigkeit der Lichtquelle, ihre Strahlungsleistung bzw. der von ihr erzeugte Lichtstrom, das Spektrum des erzeugten Lichts, ggf. die Übertragungseigenschaften eines verwendeten Lichtleitkabels und der Kopplung des Lichtleitkabels mit der Lichtquelle und dem Endoskop, die Funktionsfähigkeit der Lichtübertragung innerhalb des Endoskops, der Wirkungsgrad der Auskopplung des Lichts der Lichtquelle aus dem Endoskop, die Funktionsfähigkeit bzw. die optischen Eigenschaften des Beobachtungs-Strahlengangs im Endoskop, ggf. einschließlich eines geordneten Bündels von Lichtwellenleitern oder einer Stablinsenoptik, die Funktionsfähigkeit des Okulars oder der Kamera. Häufige Fehlerquellen bilden u.a. die einem Alterungsprozess unterworfene Lichtquelle, ggf. das Lichtleitkabel und seine Kopplung an die Lichtquelle und das Endoskop und die Kopplung einer Kamera an das Endoskop.

Insbesondere für medizinisch-diagnostische Zwecke wird Fluoreszenzlicht beobachtet. In der Photodynamischen Diagnostik (PDD) wird beispielsweise eine durch verabreichtes 5-Aminolävulinsäure (ALA) induzierte Fluoreszenz von Protoporphyrin IX beobachtet. Die Anreicherung von ALA und damit auch die Intensität der Fluoreszenz sind vom Zustand des Gewebes abhängig. Bei der Autofluoreszenz-Diagnostik (AF-Diagnostik) wird die Fluoreszenz von körpereigenen Fluorophoren beobachtet, deren Konzentration ebenfalls vom Zustand des Gewebes abhängig ist. Auch außerhalb der Medizin werden fluoreszenzdiagnostische Verfahren verwendet.

Damit remittiertes Anregungslicht bzw. Beleuchtungslicht die Fluoreszenz nicht überstrahlt, werden im Beleuchtungs- bzw. Anregungs-Strahlengang zwischen Lichtquelle und Objekt ein Beleuchtungsfilter und im Beobachtungs-Strahlengang zwischen Objekt und Kamera bzw. Okular ein Beobachtungsfilter verwendet. Das Beleuchtungsfilter ist ein Kurzpassfilter, das im wesentlichen nur die zur Anregung der Fluoreszenz erforderlichen kurzen Wellenlängen transmittiert, längere Wellenlängen hingegen überwiegend oder fast ausschließlich reflektiert oder absorbiert. Eine sehr geringe, aber nicht verschwindende Transmission im Blockbereich ist bei manchen Anwendungen erwünscht, um auch ohne Fluoreszenz ein Bild zu erhalten, das eine geringe Helligkeit aufweist aber sichtbar ist. Das Beobachtungsfilter ist ein Langpassfilter, das nur Wellenlängen der Fluoreszenz transmittiert und vom Objekt remittiertes kurzwelliges Beleuchtungslicht reflektiert oder absorbiert. Beleuchtungs- bzw. Anregungsfilter können in der Regel manuell oder maschinell ausgetauscht bzw. gewechselt werden. Beobachtungsfilter können austauschbar bzw. wechselbar sein, sind aber in vielen Fällen fest in das Endoskop eingebaut. Beispielsweise in der Urologie werden für die Beobachtung in Weißlicht, ALA- oder AF-Fluoreszenz verschiedene Endoskope verwendet, die zumindest im Beobachtungs-Strahlengang für ihre jeweilige Verwendung optimiert sind bzw. eine entsprechende Filtercharakteristik aufweisen. Zu den oben genannten Fehlerquellen bzw. Einflüssen auf die Funktionsfähigkeit des Endoskopiesystems tritt im Fall der Beobachtung von Fluoreszenz somit noch die Kombination des Beleuchtungsfilters bzw. des Spektrums der Lichtquelle einerseits und des Beobachtungsfilters andererseits.

Eine entsprechende Problemstellung existiert bei anderen optischen Untersuchungssystemen, die eine bildgebende Einrichtung und eine Lichtquelle zur optischen Untersuchung medizinischer und nicht-medizinischer Objekte in remittiertem Licht und/oder in Fluoreszenzlicht umfassen. Dazu zählen Exoskope, die beispielsweise zur Diagnostik und für mikrochirurgische Eingriffe an oder nahe Körperoberflächen verwendet werden.

In der DE 196 38 809 A1 ist eine Vorrichtung zur Prüfung und/oder Justierung eines PDD-oder PDT-Systems (PDT = Photodynamische Therapie) und/oder zur Schulung an einem derartigen System beschrieben. In einem Gehäuse ist ein Target angeordnet, dem gegenüber ein distales Ende eines Endoskops angeordnet werden kann. Die Krümmung des Targets kann der Objektfeldkrümmung einer bildgebenden Einheit des Endoskops entsprechen. Im Target sind ein Fotoelement und Lichtquellen vorgesehen. Das Fotoelement erfasst die Beleuchtungsstärke eines vom Endoskop ausgehenden Beleuchtungslichts. Eine Steuerung steuert die Lichtquellen als Funktion der vom Fotoelement erfassten Beleuchtungsstärke.

In der DE 198 55 853 A1 ist eine Vorrichtung zur Prüfung und/oder Justierung eines PDD-oder PDT-Systems und/oder zur Schulung an einem derartigen System beschrieben. Die Vorrichtung umfasst ein Lumineszenzphantom mit einem Fluoreszenzfarbstoff. Gegenüber dem Lumineszenzphantom kann ein Ende eines Endoskops angeordnet werden.

In der nachveröffentlichten DE 10 2009 043 696 sind eine Vorrichtung und ein Verfahren zum Prüfen von Endoskopen beschrieben. Die Vorrichtung umfasst ein Filtermodul mit mehreren Durchbrüchen, in denen optische Filter angeordnet sind. Das Filtermodul wird aus einer Richtung durch die Lichtquelle über ein Lichtleitkabel beleuchtet. Aus einer entgegengesetzten Richtung wird das von dem Filtermodul transmittierte Licht mittels eines Endoskops beobachtet.

Abhängig von konkreten, in der Praxis sich ergebenden Aufgabenstellungen kann jede der bislang bekannten Vorrichtungen und Verfahren Vor- und Nachteile aufweisen. Beispielsweise ermöglicht unter einigen Bedingungen und für einige Anwendungen keine der beschriebenen Vorrichtungen und Verfahren eine zuverlässige Prüfung eines optischen Untersuchungssystems darauf hin, welches Beleuchtungsfilter und welches Beobachtungsfilter vorliegen. Auch die in der DE 196 38 809 A1 und in der DE 198 55 853 A1 beschriebenen Vorrichtungen und Verfahren ermöglichen beispielsweise keine zuverlässige Unterscheidung ähnlicher Filtersätze, etwa der Filter für PDD und für AF-Diagnostik.

Eine Aufgabe der vorliegenden Erfindung besteht darin, ein verbessertes Verfahren zum Prüfen eines optischen Untersuchungssystems mit einer Lichtquelle und einer bildgebenden Einrichtung sowie einen verbesserten Referenzkörper zu schaffen.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beziehen sich auf die Prüfung eines optischen Untersuchungssystems mit einer Lichtquelle und einer bildgebenden Einrichtung zur optischen Untersuchung eines Objekts in remittiertem Licht und Fluoreszenzlicht, wobei die Lichtquelle ausgebildet ist, um Beleuchtungslicht mit einem ersten vorbestimmten Beleuchtungsspektrum oder mit einem zweiten vorbestimmten Beleuchtungsspektrum zu erzeugen, und wobei ein Beobachtungs-Strahlengang der bildgebenden Einrichtung ein erstes vorbestimmtes Transmissionsspektrum oder ein zweites vorbestimmtes Transmissionsspektrum aufweist. Beispielsweise kann das optische Untersuchungssystem entweder Beleuchtungsfilter und Beobachtungsfilter für die PDD oder Beleuchtungsfilter und Beobachtungsfilter für die AF-Diagnostik umfassen. Das Transmissionsspektrum des PDD-Beleuchtungsfilters und das Transmissionsspektrum des PDD-Beobachtungsfilters weisen einen kleinen Überlapp auf, d.h. das Produkt aus dem Transmissionsspektrum des Beleuchtungsfilters und dem Transmissionsspektrum des Beobachtungsfilters ist nur in einem kleinen Wellenlängenbereich deutlich größer als 0. Dieser kleine Wellenlängenbereich wird auch als Überlappbereich bezeichnet. Quantitativ kann der Überlappbereich definiert sein als der Wellenlängenbereich, in dem das Produkt aus den Transmissionsspektren von Beleuchtungs- und Beobachtungsfilter beispielsweise mindestens die Hälfte oder mindestens ein Drittel oder mindestens ein Zehntel seines Maximalwerts aufweist. Auch die Transmissionsspektren des Beleuchtungsfilters und des Beobachtungsfilters für die AF-Diagnostik weisen einen kleinen Überlapp auf. Der Überlappbereich liegt jedoch beim Filtersatz für die AF-Diagnostik bei größeren Wellenlängen als der Überlapp des Filtersatzes für die PDD. Die Überlappbereiche sind um etwa 25 nm gegeneinander verschoben.

Ausführungsformen der vorliegenden Erfindung beruhen auf der Idee, zum Bestimmen, welches Beleuchtungsfilter und welches Beobachtungsfilter in einem optischen Untersuchungssystem vorliegt, eine Referenzoberfläche mit einem Indikatorbereich mittels des optischen Untersuchungssystems zu beleuchten und das von der Referenzoberfläche remittierte oder aufgrund von Fluoreszenz emittierte Licht mittels des optischen Untersuchungssystems zu erfassen. Der Indikatorbereich weist eine wellenlängenabhängige optische Eigenschaft auf, die sich zwischen dem Überlappbereich des Filtersatzes für PDD und dem Überlappbereich des Filtersatzes für AF-Diagnostik wesentlich ändert. Die optische Eigenschaft ist beispielsweise der wellenlängenabhängige Remissionsgrad oder eine von der Anregungswellenlänge abhängige Fluoreszenz-Quantenausbeute.

Etwas allgemeiner formuliert, besteht eine der vorliegenden Erfindung zugrunde liegende Idee darin, zur Unterscheidung zweier Filtersätze eine Referenzoberfläche mit einem Indikatorbereich mit einer optischen Eigenschaft zu verwenden, die sich zwischen einem ersten Schwerpunkt eines ersten Produkts aus dem Transmissionsspektrum eines ersten Beleuchtungsfilters und dem Transmissionsspektrum eines ersten Beobachtungsfilters und dem zweiten Schwerpunkt eines zweiten Produkts aus einem Transmissionsspektrum eines zweiten Beleuchtungsfilters und einem Transmissionsspektrum eines zweiten Beobachtungsfilters wesentlich ändert. Die Schwerpunkte der Produkte können ohne Wichtung der Wellenlängen oder beispielsweise mit Wichtung der Wellenlängen nach der spektralen Empfindlichkeit des menschlichen Auges oder einer Kamera berechnet werden. Anstelle von Beleuchtungsfiltern, die ein ursprünglich breites, insbesondere weißes, Spektrum einer Lichtquelle modifizieren, können Lichtquellen verwendet werden, die ohne Verwendung weiterer Filter die gewünschten Beleuchtungsspektren erzeugen, beispielsweise schmalbandige Leuchtdioden, Laserdioden bzw. Halbleiterlaser, andere Laser, Gasentladungslampen etc. Eine wesentliche Änderung liegt beispielsweise vor, wenn der Remissionsgrad oder die Fluorzeszenz-Quantenausbeute an den beiden Schwerpunkten Werte annimmt, die sich mindestens wie 3:2 oder 2:1 oder 3:1 unterscheiden.

Ein Verfahren bezieht sich auf ein optisches Untersuchungssystem mit einer Lichtquelle und einer bildgebenden Einrichtung zur optischen Untersuchung eines Objekts in remittiertem Licht und Fluoreszenzlicht, wobei die Lichtquelle ausgebildet ist, um Beleuchtungslicht zumindest entweder mit einem ersten vorbestimmten Beleuchtungsspektrum oder mit einem zweiten vorbestimmten Beleuchtungsspektrum zu erzeugen, und wobei ein Beobachtungs-Strahlengang der bildgebenden Einrichtung zumindest entweder ein erstes vorbestimmtes Transmissionsspektrum oder ein zweites vorbestimmtes Transmissionsspektrum aufweist. Bei dem Verfahren wird die bildgebende Einrichtung gegenüber einer Referenzoberfläche mit einem Indikatorbereich mit einer wellenlängenabhängigen optischen Eigenschaft angeordnet, wobei sich die optische Eigenschaft zwischen einem ersten Schwerpunkt eines ersten Produkts aus dem ersten vorbestimmten Beleuchtungsspektrum und dem ersten vorbestimmten Transmissionsspektrum und einem zweiten Schwerpunkt eines zweiten Produkts aus dem zweiten vorbestimmten Beleuchtungsspektrum und dem zweiten vorbestimmten Transmissionsspektrum wesentlich ändert. Die Referenzoberfläche wird mit Beleuchtungslicht der Lichtquelle beleuchtet, und mittels der bildgebenden Einrichtung wird ein Bild der Referenzoberfläche erfasst. Anhand des erfassten Bilds wird bestimmt, welches Beleuchtungsspektrum und welches Transmissionsspektrum im Beobachtungs-Strahlengang beim Erfassen des Bilds vorlagen.

Das Verfahren ist insbesondere anwendbar auf ein Endoskopiesystem mit einer Lichtquelle und einem Endoskop für medizinische oder nicht-medizinische Anwendungen und insbesondere für die Fluoreszenz-Diagnostik. Das optische Untersuchungssystem kann alternativ eine von mehreren alternativen Lichtquellen, die jeweils unterschiedliche Beleuchtungsspektren erzeugen, umfassen oder eine Lichtquelle, deren Beleuchtungsspektrum durch maschinellen oder manuellen Austausch eines Beleuchtungsfilters verändert werden kann. Das optische Untersuchungssystem kann alternativ eine von mehreren bildgebenden Einrichtungen, deren Beobachtungs-Strahlengänge unterschiedliche Transmissionsspektren aufweisen, oder eine bildgebende Einrichtung mit einem austauschbaren Beobachtungsfilter aufweisen. Die Referenzoberfläche wird insbesondere mittels eines in die bildgebende Einrichtung integrierten Beleuchtungs-Strahlengangs beleuchtet. Das Bild der Referenzoberfläche kann über ein Okular durch das menschliche Auge oder mittels einer Kamera erfasst werden.

Der Indikatorbereich kann einen beliebigen Flächenanteil an der Referenzoberfläche haben. Insbesondere kann der Indikatorbereich als räumlich begrenzte Marke an einer insgesamt deutlich größeren Referenzoberfläche vorliegen oder die gesamte Referenzoberfläche einnehmen. Im erstgenannten Fall ist der Indikatorbereich beispielsweise in Form eines oder mehrerer alphanumerischer Zeichen, eines Piktogramms oder eines anderen Symbols gestaltet.

Das hier beschrieben Prüfverfahren ermöglicht auf erstaunlich einfache Weise eine Prüfung eines optischen Untersuchungssystems, insbesondere hinsichtlich des vorliegenden Beleuchtungsspektrums bzw. Beleuchtungsfilters und hinsichtlich des vorliegenden Beobachtungsfilters. Dabei ist durch eine rein qualitative oder höchstens semiquantitative Bewertung des erfassten Bilds der Referenzoberfläche mit dem Indikatorbereich nicht nur eine Identifizierung fehlerhafter Filterkombinationen, sondern auch eine Unterscheidung zulässiger Filterkombinationen für die PDD und die AF-Diagnostik möglich. Ob ein PDD-Beleuchtungsfilter mit einem PDD-Beobachtungsfilter oder ein AF-Beleuchtungsfilter mit einem AF-Beobachtungsfilter kombiniert ist, ist mit bloßem Auge ohne Verwendung des hier vorgeschlagenen Indikatorbereichs in der Regel nicht möglich. Auch ist bei der PDD nicht oder nur für sehr erfahrenes medizinisches Personal erkennbar, wenn das optische Untersuchungssystem irrtümlich Beleuchtungs- und Beobachtungsfilter aufweist, die für die AF-Diagnostik vorgesehen sind. Entsprechendes gilt für eine irrtümliche Verwendung eines optischen Untersuchungssystems mit Beleuchtungs- und Beobachtungsfilter für PDD in der AF-Diagnostik. Gleichzeitig liefert jedoch eine PDD oder eine AF-Diagnostik mit dem falschen Filtersatz in vielen Fällen ein unzutreffendes Ergebnis. Ein unzutreffender Befund kann schwerste Folgen haben. Sicherzustellen, dass beispielsweise bei der PDD oder der AF-Diagnostik die jeweils richtigen Beleuchtungs- und Beobachtungsfilter eingesetzt werden, ist somit von herausragender Bedeutung in der Qualitätssicherung.

Das hier beschriebene Prüfverfahren ist in vielen Varianten für optische Untersuchungssysteme ohne Kamera geeignet, da bereits mit bloßem Auge am Okular der bildgebenden Einrichtung anhand des Bilds der Referenzoberfläche erkannt werden kann, welches Beleuchtungsfilter und welches Beobachtungsfilter in dem optischen Untersuchungssystem vorliegen. Das Prüfverfahren ist somit gerade auch für jene einfacheren optischen Untersuchungssysteme ohne Kamera geeignet, die von medizinischem Personal immer noch häufig bevorzugt werden.

Beim Bestimmen des Beleuchtungsspektrums bzw. des Beleuchtungsfilters und des Transmissionsspektrums im Beobachtungs-Strahlengang bzw. des Beobachtungsfilters kann das mittels der bildgebenden Einrichtung des vorliegenden optischen Untersuchungssystems erfasste Bild mit einem Referenzbild, insbesondere mit mehreren Referenzbildern, verglichen werden. Beispielsweise werden Referenzbilder bereitgestellt, die den verschiedenen möglichen Kombinationen aus Beleuchtungsfilter und Beobachtungsfilter entsprechen. Bei einem Vergleich des erfassten Bilds mit den Referenzbildern kann mit geringem Aufwand, schnell und zuverlässig festgestellt werden, welchem Referenzbild das erfasste Bild am ähnlichsten ist.

Alternativ oder zusätzlich kann im erfassten Bild die Wiedergabe des Indikatorbereichs mit der Wiedergabe eines Referenzbereichs auf der Referenzoberfläche verglichen werden. Der Indikatorbereich und der Referenzbereich sind beispielsweise so ausgebildet, dass anhand eines Vergleichs der Helligkeiten und/oder der Farbtöne des Indikatorbereichs und des Referenzbereichs das Beleuchtungsspektrum und das Transmissionsspektrum im Beobachtungs-Strahlengang identifiziert werden können. Insbesondere wird der Indikatorbereich mit mehreren Referenzbereichen verglichen. Ein Vergleich der Wiedergabe des Indikatorbereichs mit der Wiedergabe von einem oder mehreren Referenzbereichen im erfassten Bild ist im Moment des Erfassens des Bilds, insbesondere mit dem Auge am Okular der bildgebenden Einrichtung, möglich. Bei einer Gestaltung des Indikatorbereichs und/oder des oder der Referenzbereiche in Form von alphanumerischen Zeichen, Piktogrammen oder anderen Symbolen wird das Risiko einer fehlerhaften Bestimmung des Beleuchtungsspektrums und des Transmissionsspektrums im Beobachtungs-Strahlengang weiter reduziert.

Die optische Eigenschaft des Indikatorbereichs und die entsprechende optische Eigenschaft des Referenzbereichs können sich zwischen dem ersten Schwerpunkt und dem zweiten Schwerpunkt gegenläufig ändern. Dies hat beispielsweise zur Folge, dass im erfassten Bild die Wiedergabe des Indikatorbereichs heller ist als die Wiedergabe des Referenzbereichs, wenn das optische Untersuchungssystem Beleuchtungsfilter und Beobachtungsfilter für die PDD aufweist, und die Wiedergabe des Referenzbereichs heller als die Wiedergabe des Indikatorbereichs ist, wenn das optische Untersuchungssystem Beleuchtungs- und Beobachtungsfilter für die AF-Diagnostik aufweist.

Der Indikatorbereich umfasst insbesondere ein Indikatorfilter, dessen Filterkante bzw. Flanke zwischen dem ersten Schwerpunkt und dem zweiten Schwerpunkt liegt. Das Indikatorfilter ist beispielsweise eine wellenlängenabhängig absorbierende Schicht vor einer reflektierenden Fläche oder ein wellenlängenabhängig reflektierendes Filter. Hinter bzw. unter dem Indikatorfilter bzw. an der von der bildgebenden Einrichtung abgewandten Seite des Indikatorfilters kann der Indikatorbereich eine fluoreszierende Fläche umfassen. In diesem Fall ist das Indikatorfilter insbesondere ein Transmissionsfilter, das bei Wellenlängen, die über der Filterkante liegen, einen hohen Transmissionsgrad und bei Wellenlängen, die unter der Filterkante liegen, einen hohen Reflexionsgrad aufweist. Zusammen mit der Farbverschiebung durch die fluoreszierende Fläche ist so ein Farbumschlag des Indikatorbereichs an der Filterkante des Indikatorfilters realisierbar. Beispielsweise liegt die Filterkante des Indikatorfilters bei 440 nm bis 450 nm, und Fluoreszenz im grünen und/oder roten Wellenlängenbereich der fluoreszierenden Fläche unter dem Indikatorfilter kann durch Wellenlängen im Bereich bis ca. 460 nm angeregt werden. Dann erscheint der Indikatorbereich bei Beleuchtung mit einem PDD-Beleuchtungsspektrum und Beobachtung mittels eines PDD-Beobachtungsfilters blau und bei Beleuchtung mittels eines AF-Beleuchtungsspektrums und Beobachtung durch ein AF-Beobachtungsfilter grün bis rot. Die beiden Filtersätze können somit anhand der Farbe der Wiedergabe des Indikatorbereichs im erfassten Bild unterschieden werden.

Als Indikatorfilter kommen insbesondere Filter in Betracht, die den Beleuchtungs- und Beobachtungsfiltern für PDD oder für AF-Diagnostik entsprechen oder ähnlich sind. Der Reflexionsgrad R (λ) und der Transmissiongrad T(λ) verhalten sich in vielen Fällen zumindest näherungsweise komplementär, R (λ) = 1-T (λ).

Das Bild der Referenzoberfläche kann unmittelbar visuell über ein Okular oder durch eine Kamera erfasst werden. Wie bereits erwähnt, ermöglicht die vorliegende Erfindung eine zuverlässige Verifizierung der richtigen Filterkombination auch an einem einfachen optischen Untersuchungssystem ohne Kamera. Die Verwendung einer Kamera schafft zusätzlich die Möglichkeit einer zumindest semiquantitativen Auswertung des erfassten Bilds.

Wenn das optische Untersuchungssystem eine Kamera umfasst, kann vor dem Erfassen des Bilds der Referenzoberfläche durch die Kamera ein Betriebszustand der Kamera auf einen vorbestimmten Wert eingestellt werden. Beispielsweise werden die Belichtungszeit und/oder die Verstärkung und/oder Weißabgleich-Parameter der Kamera auf vorbestimmte Werte eingestellt. Alternativ oder zusätzlich kann der während des Erfassens des Bilds durch die Kamera vorliegende Betriebszustand der Kamera erfasst werden. Beispielsweise werden von der Kamera oder einer Kamerasteuerung in Anpassung an die Belichtungssituation automatisch eingestellte Parameter wie die Belichtungszeit, die Verstärkung und Weißabgleich-Parameter, erfasst, insbesondere soweit sie nicht zuvor auf vorbestimmte Werte eingestellt wurden. Die Erfassung des Betriebszustands der Kamera ermöglicht Rückschlüsse auf die Helligkeit und die spektralen Eigenschaften des durch die Kamera mittels der bildgebenden Einrichtung erzeugten Bilds der Referenzoberfläche.

Bei jedem der hier beschriebenen Verfahren können ferner Patientendaten erfasst und Information über das vorliegende Beleuchtungsspektrum und das vorliegende Transmissionsspektrum im Beobachtungs-Strahlengang des optischen Untersuchungssystems und die Patientendaten in einer Datenbank abgelegt werden. Im Fall eines Erfassens des Bilds der Referenzoberfläche unmittelbar durch das menschliche Auge an einem Okular der bildgebenden Einrichtung, erfolgt dies insbesondere nachdem medizinisches Personal das Ergebnis der oben beschriebenen Prüfung an einer Benutzerschnittstelle eingegeben hat. Bei Erfassen des Bilds mittels einer Kamera und automatischer Auswertung des erfassten Bilds, beispielsweise durch die Kamerasteuerung, kann dies ohne Eingriff des medizinischen Personals selbständig erfolgen.

Durch Integration des Prüfverfahrens mit dem Erfassen und Speichern der Patientendaten kann sichergestellt werden, dass bei jeder medizinisch-diagnostischen Verwendung des optischen Untersuchungssystems dieses auch hinsichtlich seiner Funktionsfähigkeit und/oder einer anderen Eigenschaft geprüft und das Ergebnis der Prüfung dokumentiert wird. Das Prüfverfahren kann damit zu einem zuverlässigen und nicht manipulierbaren Bestandteil der Qualitätssicherung im klinischen Alltag werden.

Bei Erfassen des Bilds durch eine Kamera kann nach der oben beschriebenen Prüfung eine Meldung erzeugt werden. Diese Meldung nennt das bestimmte Beleuchtungsspektrum bzw. das Beleuchtungsfilter und das bestimmte Transmissionsspektrum des Beobachtungs-Strahlengangs bzw. das Beobachtungsfilter des vorliegenden Untersuchungssystems. Alternativ oder zusätzlich kann die Meldung eine Handlungsanweisung oder eine Handlungsempfehlung enthalten. Beispielsweise enthält die Meldung eine Aufforderung, das Beleuchtungsfilter oder die Lichtquelle oder das Beobachtungsfilter oder die bildgebende Einrichtung auszutauschen und die Prüfung des optischen Untersuchungssystems anschließend zu wiederholen.

Die vorliegende Erfindung ist als Verfahren oder als Computer-Programm mit Programmcode zur Durchführung oder Steuerung eines solchen Verfahrens, wenn das Computer-Programm auf einem Computer oder einem Prozessor abläuft, implementierbar. Ferner ist die Erfindung als Computer-Programm-Produkt mit auf einem maschinenlesbaren Träger (beispielsweise einem ROM-, PROM-, EPROM-, EEPROM- oder Flash-Speicher, einer CD-ROM, DVD, HD-DVD, Blue-Ray-DVD, Diskette oder Festplatte) oder in Form von Firmware gespeichertem Programmcode zur Durchführung von einem der genannten Verfahren, wenn das Computer-Programm-Produkt auf einem Computer, Rechner oder Prozessor abläuft, implementierbar. Ferner kann die vorliegende Erfindung als digitales Speichermedium (beispielsweise ROM-, PROM-, EPROM-, EEPROM- oder Flash-Speicher, CD-ROM, DVD, HD-DVD, Blue-Ray-DVD, Diskette oder Festplatte) mit elektronisch auslesbaren Steuersignalen, die so mit einem programmierbaren Computer-oder Prozessor-System zusammenwirken können, dass eines der beschriebenen Verfahren ausgeführt wird, implementiert werden.

Ferner kann die vorliegende Erfindung als Steuerung für ein optisches Untersuchungssystem mit einer bildgebenden Einrichtung, insbesondere einem Endoskop, einer Kamera und einer Lichtquelle zur optischen Untersuchung eines Objekts implementiert werden, wobei die Steuerung ausgebildet ist, um eines der beschriebenen Verfahren auszuführen, oder wobei die Steuerung ein Computer-Programm, ein Computer-Programm-Produkt oder ein digitales Speichermedium umfasst, wie sie im vorangehenden Absatz beschrieben wurden. Ein Referenzkörper zum Prüfen eines optischen Untersuchungssystems mit einer Lichtquelle zum Erzeugen eines ersten vorbestimmten Beleuchtungsspektrums oder eines zweiten vorbestimmten Beleuchtungsspektrums und einer bildgebenden Einrichtung mit einem Beobachtungs-Strahlengang mit einem ersten vorbestimmten Transmissionsspektrum oder einem zweiten vorbestimmten Transmissionsspektrum umfasst eine Referenzoberfläche und einen Indikatorbereich an der Referenzoberfläche, wobei eine wellenlängenabhängige optische Eigenschaft des Indikatorbereichs sich zwischen einem ersten Schwerpunkt eines ersten Produkts aus dem ersten vorbestimmten Beleuchtungsspektrum und dem ersten vorbestimmten Transmissionsspektrum und einem zweiten Schwerpunkt eines zweiten Produkts aus dem zweiten vorbestimmten Beleuchtungsspektrum und dem zweiten vorbestimmten Transmissionsspektrum wesentlich ändert.

Der Referenzkörper ist insbesondere zur Durchführung eines der oben beschriebenen Verfahren ausgebildet. Insbesondere weist der Indikatorbereich ein Indikatorfilter auf, wie es oben beschrieben wurde, wobei unter dem Indikatorfilter eine fluoreszierende Fläche angeordnet sein kann. Ferner kann die Referenzoberfläche einen Referenzbereich, insbesondere mehrere Referenzbereiche, aufweisen.

### Kurzbeschreibung der Figuren

Nachfolgend werden Ausführungsformen anhand der beigefügten Figuren näher erläutert. Es zeigen:
- Figur 1: eine schematische Darstellung eines optischen Untersuchungssystems;
- Figur 2: eine schematische Darstellung eines Endoskops mit einer Prüfvorrichtung;
- Figur 3: eine schematische Darstellung mehrerer Spektren;
- Figur 4: eine schematische Darstellung weiterer Spektren;
- Figur 5: eine schematische Darstellung von Produkten von Transmissionsspektren;
- Figur 6: eine schematische Darstellung weiterer Transmissionsspektren;
- Figur 7: eine schematische Darstellung eines Flussdiagramms.

### Beschreibung der Ausführungsformen

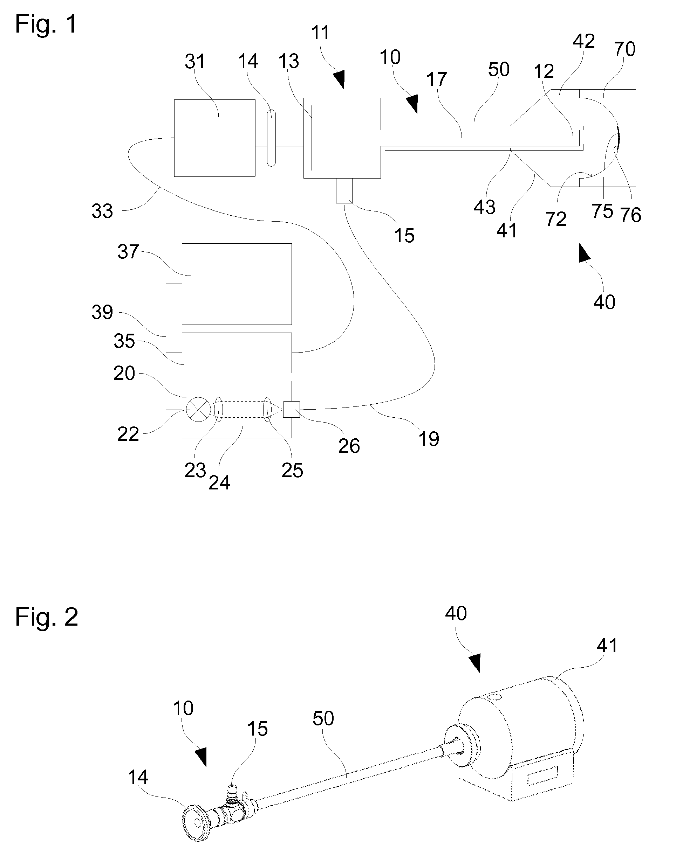

Figur 1 zeigt eine schematische Darstellung eines optischen Untersuchungssystems. Das optische Untersuchungssystem ist in diesem Beispiel ein Endoskopiesystem, das beispielsweise bei medizinisch-diagnostischen Verfahren in der Urologie und in anderen Fachgebieten einsetzbar ist. Das Endoskopiesystem umfasst ein Endoskop 10 mit einem proximalen Ende 11 und einem distalen Ende 12. Das Endoskop 10 umfasst einen Beleuchtungs- bzw. Anregungs-Strahlengang und einen Beobachtungs-Strahlengang, die in Figur 1 nicht im Detail dargestellt sind. Der Beleuchtungs-Strahlengang umfasst insbesondere einen oder mehrere Lichtwellenleiter zum Übertragen von Beleuchtungs- bzw. Anregungslicht vom proximalen Ende 11 zum distalen Ende 12 und einen Lichtaustritt am distalen Ende 12, durch den Beleuchtungslicht aus dem distalen Ende 12 des Endoskops 10 austreten kann, um ein zu betrachtendes Objekt zu beleuchten. Der Beobachtungs-Strahlengang umfasst einen Lichteintritt am distalen Ende 12 des Endoskops 10, eine Optik zum Übertragen von Beobachtungslicht, das von einem betrachteten Objekt ausgeht, vom distalen Ende 12 zum proximalen Ende 11, ein Beobachtungsfilter 13 und ein Okular 14. Zum Übertragen des Beobachtungslichts vom distalen Ende 12 zum proximalen Ende 11 des Endoskops 10 ist beispielsweise eine Stablinsenoptik oder ein geordnetes Bündel von Lichtwellenleitern in einem Schaft 17 des Endoskops 10 vorgesehen. Das Endoskop 10 weist ferner am proximalen Ende 11 eine Kupplung 15 zum mechanischen und optischen Koppeln eines Lichtleitkabels 19 mit dem beschriebenen Beleuchtungs-Strahlengang im Endoskop 10 auf.

Das Endoskop 10 ist über das Lichtleitkabel 19 mit einer Lichtquellenvorrichtung 20 gekoppelt. Die Lichtquellenvorrichtung 20 umfasst eine Lichtquelle 22, beispielsweise eine Halogen-Glühlampe, eine Hochdruck-Gasentladungslampe, eine Leuchtdiode oder einen Laser. Ferner umfasst die Lichtquellenvorrichtung 20 eine erste Sammellinse 23, ein Beleuchtungsfilter 24 und eine zweite Sammellinse 25. Die Lichtquelle 22 ist über die erste Sammellinse 23, das Beleuchtungsfilter 24, die zweite Sammellinse 25 und eine Kupplung 26 mit dem Lichtleitkabel 19 gekoppelt.

Eine Kamera 31 ist über das Okular 14 mit dem Endoskop 10 und dessen Beobachtungs-Strahlengang mechanisch bzw. optisch gekoppelt. Die Kamera 31 umfasst einen lichtempfindlichen Bildsensor, beispielsweise einen CCD- oder CMOS-Sensor, zum Wandeln von auf den Bildsensor fallendem Licht in analoge oder digitale elektrische Signale. Die Kamera 31 ist über ein Signalkabel 33 zur Übertragung analoger oder digitaler elektrischer oder optischer Signale mit einer Kamerasteuerung 35 gekoppelt, die auch als CCU = camera control unit bezeichnet wird.

Die Lichtquellenvorrichtung 20, die Kamerasteuerung 35 und ein Bildschirm 37 sind über einen Kommunikationsbus 39 oder mehrere separate Signalleitungen miteinander gekoppelt. Über den Kommunikationsbus 39 können weitere, in Figur 1 nicht dargestellte Vorrichtungen innerhalb oder außerhalb des Behandlungsraums, in dem das Endoskopiesystem angeordnet ist, mit der Lichtquellenvorrichtung 20, der Kamerasteuerung 35 und dem Bildschirm 37 gekoppelt sein, beispielsweise eine Datenbank, eine Tastatur, eine Computermaus und andere Benutzerschnittstellen.

In Figur 1 ist ferner eine Prüfvorrichtung 40 mit einem lichtdichten Gehäuse 41, einem Hohlraum 42 in dem lichtdichten Gehäuse 41 und einer Öffnung 43 zum Hohlraum 42 dargestellt. Das distale Ende 12 des Endoskops 10 ist durch die Öffnung 43 in den Hohlraum 42 der Prüfvorrichtung 40 eingeführt. Eine in der Öffnung 43 angeordnete Positionierungseinrichtung 50 hält form- und/oder kraftschlüssig den Schaft 17 des Endoskops 10 so, dass das distale Ende 12 des Endoskops 10 an einer vorbestimmten Position und in einer vorbestimmten Richtung im Hohlraum 42 angeordnet ist. Ferner verhindert die Positionierungseinrichtung 50, zumindest wenn der Schaft 17 des Endoskops 10 in der Positionierungseinrichtung 50 angeordnet ist, weitgehend das Eindringen von Licht aus der Umgebung durch die Öffnung 43 in den Hohlraum 42 im Gehäuse 41.

Im Hohlraum 42 der Prüfvorrichtung 40 ist ferner ein Referenzkörper 70 mit einer Referenzoberfläche 72 angeordnet. Die Referenzoberfläche 72 weist vorbestimmte optische Eigenschaften und die räumliche Gestalt eines Ausschnitts aus einer Kugeloberfläche oder aus einem Kreiszylindermantel auf. Die für das distale Ende 12 des Endoskops 10 vorgesehene Position liegt insbesondere am Mittelpunkt dieser Kugeloberfläche bzw. an der Symmetrieachse des Kreiszylindermantels. Insbesondere liegt der objektseitige Hauptpunkt bzw. der Schnittpunkt der optischen Achse mit der objektseitigen Hauptebene der bildgebenden Einrichtung 10 am Mittelpunkt der Kugeloberfläche bzw. an der Symmetrieachse des Kreiszylindermantels.

Die Referenzoberfläche 72 weist mehrere Bereiche mit unterschiedlichen, jeweils zeitlich unveränderliche bzw. stabile vorbestimmte optische Eigenschaften auf. Ein Bereich der Referenzoberfläche 72 kann weiß sein bzw. einen Remissionsgrad aufweisen, der im für das menschliche Auge sichtbaren Spektralbereich im Wesentlichen wellenlängenunabhängig ist. Alternativ kann ein Bereich der Referenzoberfläche 72 überwiegend farbig sein bzw. einen im für das menschliche Auge sichtbaren Spektralbereich wellenlängenabhängigen Remissionsgrad aufweisen. Alternativ oder zusätzlich kann ein Bereich der Referenzoberfläche 72 überwiegend fluoreszierend sein. Dabei liegen die zur Anregung der Fluoreszenz erforderlichen Wellenlängen beispielsweise im ultravioletten oder, für medizinische Anwendungen bevorzugt, im blauen Spektralbereich und das emittierte Fluoreszenzlicht im grünen, roten oder infraroten Spektralbereich. Bei dem in Figur 1 dargestellten Beispiel ist die Referenzoberfläche 72 überwiegend weiß.

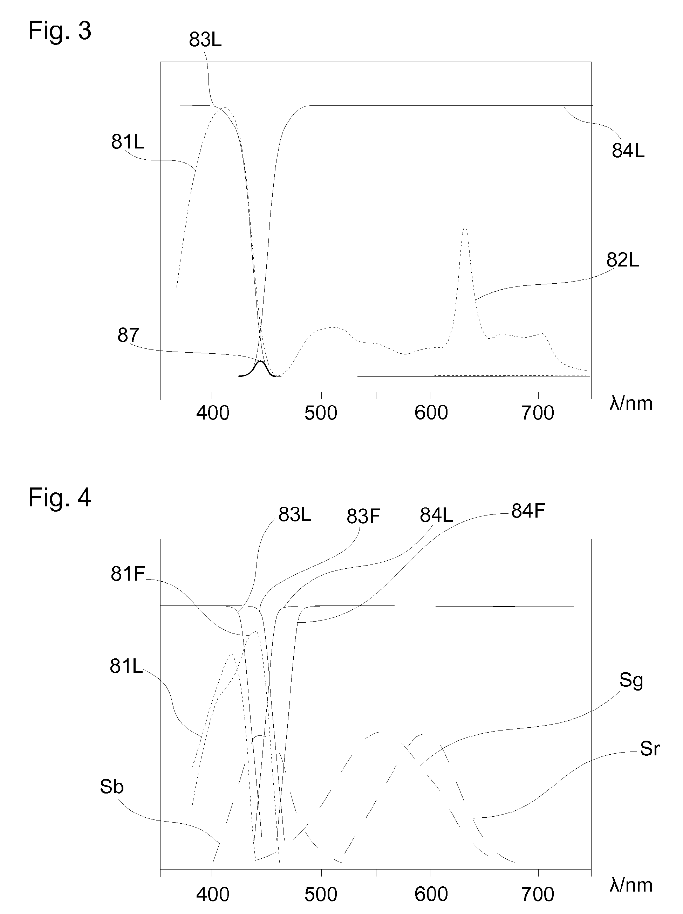

Die Referenzoberfläche 72 weist einen Indikatorbereich 73 und einen Referenzbereich 75 auf, die jeweils vom Rest der Referenzoberfläche 72 verschiedene optische Eigenschaften aufweisen. Der Indikatorbereich 75 und der Referenzbereich 76 können mit scharfen Rändern oder aufgrund ihrer Anordnung oder Gestalt eine Fokussierung oder eine Einstellung der Brennweite bzw. der Größe des Sichtfelds der bildgebenden Einrichtung vereinfachen oder ermöglichen. Darüber hinaus weisen der Indikatorbereich 75 und der Referenzbereich 76 optische Eigenschaften auf, die eine Bestimmung des Transmissionsspektrums des Beleuchtungsfilters 24 und des Transmissionsspektrums des Beobachtungsfilters 13 vereinfachen. Dazu weisen der Indikatorbereich 75 und der Referenzbereich 76 jeweils einen wellenlängenabhängigen Remissionsgrad auf. Dies wird unten anhand der Figuren 3 bis 6 näher beschrieben.

Der Referenzkörper 70 besteht, von dem Indikatorbereich 75 und dem Referenzbereich 76 an der Referenzoberfläche 72 abgesehen, insbesondere aus Polytetrafluorethylen PTFE, das beispielsweise unter dem Markennamen Teflon von DuPont vertrieben wird, oder aus Silikon. Sowohl PTFE als auch Silikon kann mit weißen oder farbigen Pigmenten oder Farbstoffen gefüllt sein.

Figur 2 zeigt eine schematische axonometrische Darstellung eines Endoskops 10 und einer Prüfvorrichtung 40, die dem Endoskop und der Prüfvorrichtung ähnlich sind, die oben anhand der Figur 1 dargestellt wurden. Im Unterschied zur Figur 1 sind keine separate Lichtquelle, Kamera oder andere Vorrichtungen gezeigt. Die exakte Positionierung des distalen Endes des Endoskops 10 in der Prüfvorrichtung 40 wird bei diesem Beispiel durch Formschluss zwischen der Positionierungseinrichtung 50 und dem distalen Ende 11 des Endoskops 10 erreicht, insbesondere mittels eines mechanischen Anschlags und/oder einer Rastverbindung.

Die nachfolgend beschriebenen Prüfverfahren sind auch auf optische Untersuchungssysteme und Prüfvorrichtungen anwendbar, die sich von den oben anhand der Figuren 1 und 2 dargestellten unterscheiden. Beispielsweise sind die Prüfverfahren unabhängig davon anwendbar, ob eine Lichtquelle und/oder eine Kamera als separate und mit dem Endoskop koppelbare Einheiten ausgeführt oder in das Endoskop an dessen proximalem oder distalem Ende integriert sind. Ferner sind die Prüfverfahren anwendbar, wenn das Anregungs- bzw. Beleuchtungslicht nicht über das Endoskop bzw. allgemeiner über die bildgebende Einrichtung, sondern auf andere Weise auf das zu betrachtende Objekt bzw. die Referenzoberfläche geleitet wird. Auch die Anordnung von Beleuchtungs- und Beobachtungsfiltern kann von den oben anhand der Figuren 1 und 2 dargestellten Beispielen abweichen. Um das Verständnis zu vereinfachen, werden nachfolgend dennoch beispielhaft Bezugszeichen aus den Figuren 1 und 2 verwendet.

Das nachfolgend beschriebenen Prüfverfahrens ist insbesondere anwendbar, wenn die vorgesehene Anwendung des optischen Untersuchungssystems PDD, AF-Diagnostik oder eine andere Fluoreszenz-Diagnostik ist. Zur Erläuterung werden zunächst Fluoreszenz-Anregungs- und -Abregungsspektren sowie Transmissionsspektren von Beleuchtungs- und Beobachtungsfiltern für die Fluoreszenz-Diagnostik beschrieben. Beispielsweise die zur PDD und die zur AF-Diagnostik verwendeten Filter unterscheiden sich, sind jedoch bei visueller Betrachtung leicht verwechselbar. Das oben beschriebene Prüfverfahren kann so modifiziert werden, dass die verwendeten Filter identifiziert werden können.

Figur 3 zeigt eine schematische Darstellung eines Fluoreszenz-Anregungsspektrums 81L und eines Fluoreszenz-Abregungsspektrums 82L von durch 5-Aminolävulinsäure (ALA) induzierter Fluoreszenz von Protoporphyrin IX. Der Abszisse ist die Wellenlänge λ zugeordnet, der Ordinate die Quantenausbeute bzw. die Intensität in willkürlichen Einheiten. Ferner sind ein Transmissionsspektrum 83L eines geeigneten Beleuchtungsfilters 24 und ein Transmissionsspektrum 84L eines geeigneten Beobachtungsfilters 13 dargestellt. Für die Transmissionsspektren 83L und 84L ist der Ordinate jeweils der Transmissionsgrad zugeordnet.

Ferner ist das Produkt 87 aus den Transmissionsspektren 83L, 84L bzw. das Transmissionsspektrum der hintereinander geschalteten Beleuchtungs- und Beobachtungsfilter dargestellt. Die Filterkanten des Beleuchtungsfilters 24 und des Beobachtungsfilters 23 sind so gewählt, dass das Produkt aus deren Transmissionsspektren in einem kleinen Wellenlängenbereich nicht 0 ist, der auch als Überlappbereich bezeichnet wird. Ein kleiner Anteil des auf das beobachtete Objekt treffenden Beleuchtungslichts kann deshalb durch das Beobachtungsfilter 13 beobachtet werden. Das beobachtete Objekt ist deshalb auch ohne Fluoreszenz in (ohne Wellenlängenverschiebung) remittiertem blauem Beleuchtungslicht erkennbar. Die Fluoreszenz erscheint demgegenüber überwiegend im grünen und roten Spektralbereich. Damit liegt zwischen fluoreszierenden und nicht-fluoreszierenden Bereichen eines mittels des optischen Untersuchungssystems beobachteten Objekts ein deutlicher Farbkontrast vor.

Figur 4 zeigt eine schematische Darstellung von Fluoreszenz-Anregungsspektren sowie Transmissionsspektren von Beleuchtungs- und Beobachtungsfiltern, die für verschiedene Arten der Fluoreszenz-Diagnostik verwendet werden. Der Abszisse ist die Wellenlänge λ zugeordnet. Gezeigt sind neben dem Fluoreszenz-Anregungsspektrum 81L, dem Transmissionsspektrum 83L des Beleuchtungsfilters und dem Transmissionsspektrum 84L des Beobachtungsfilters für PDD auch das Fluoreszenz-Anregungsspektrum 81F, das Transmissionsspektrum 83F des Beleuchtungsfilters und das Transmissionsspektrum 84F des Beobachtungsfilters zur Beobachtung von Autofluoreszenz (AF) von Gewebe.

In Figur 4 sind ferner die spektralen Empfindlichkeiten Sb, Sg, Sr der blauen, grünen und roten Farbrezeptoren des menschlichen Auges dargestellt. Da Kameras möglichst weitgehend an das Farbempfinden des menschlichen Auges angepasst werden, weisen sie in der Regel ähnliche spektrale Empfindlichkeiten auf oder trennen die Farbkanäle noch schärfer. Im Vergleich der Transmissionsspektren 83L, 83F, 84L, 84F der Beleuchtungs- und Beobachtungsfilter für PDD und AF mit den spektralen Empfindlichkeiten der Farbrezeptoren des menschlichen Auges wird deutlich, dass die geringen Unterschiede zwischen den Transmissionsspektren der Beleuchtungs- und Beobachtungsfilter für PDD und AF für das menschliche Auge nur unter guten Bedingungen im unmittelbaren Vergleich - der selten möglich ist ― erkennbar sind.

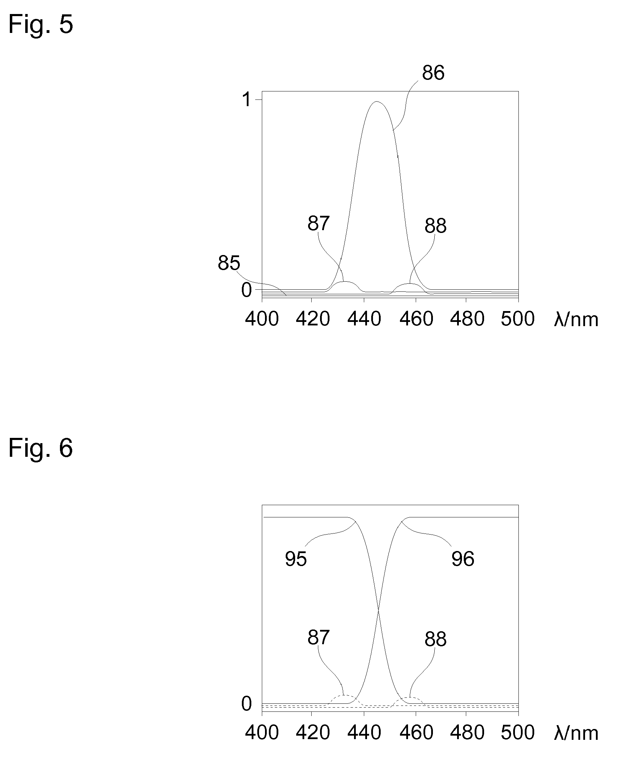

Figur 5 zeigt eine schematische Darstellung verschiedener Produkte jeweils eines Transmissionsspektrums eines Beleuchtungsfilters und eines Transmissionsspektrums eines Beobachtungsfilters. Die Kurven sind vertikal geringfügig gegeneinander verschoben, damit sie leichter unterschieden werden können. Tatsächlich sind alle Produkte bei Wellenlängen um 400 nm und bei Wellenlängen um 500 nm näherungsweise 0.

Das Produkt 85 aus dem Transmissionsspektrum 83L des PDD-Beleuchtungsfilters und dem Transmissionsspektrum 84F des AF-Beobachtungsfilters ist für alle Wellenlängen sehr klein bzw. näherungsweise 0. Das AF-Beobachtungsfilter ist somit für remittiertes PDD-Anregungslicht nicht transparent.

Das Produkt 86 aus dem Transmissionsspektrum 83F des Beleuchtungsfilters für AF-Diagnostik und dem Transmissionsspektrum 84L des Beobachtungsfilters für PDD ist für Wellenlängen im Bereich von ca. 430 nm bis ca. 460 nm deutlich größer als 0. Das PDD-Beobachtungsfilter ist für remittiertes AF-Anregungslicht somit in einem deutlich sichtbaren Maß transparent.

Das Produkt 87 aus dem Transmissionsspektrum 83L des Beleuchtungsfilters für PDD und dem Transmissionsspektrum 84L des Beobachtungsfilters für PDD ist, wie bereits oben anhand der Figur 4 dargestellt, in einem kleinen Wellenlängenbereich zwischen ca. 430 nm und ca. 440 nm nicht 0. Das PDD-Beobachtungsfilter ist für remittiertes PDD-Anregungslicht geringfügig transparent.

Das Produkt 88 aus dem Transmissionsspektrum 83F des Beleuchtungsfilters für AF und dem Transmissionsspektrum 84F des Beobachtungsfilters für AF ist in einem kleinen Wellenlängenbereich in der Gegend von 460 nm nicht 0. Das AF-Beobachtungsfilter ist für remittiertes AF-Anregungslicht geringfügig transparent.

Bei Betrachtung einer weißen, nicht fluoreszierenden Referenzoberfläche mit einem optischen Untersuchungssystem kann somit unter günstigen Umständen unterscheidbar sein, ob ein PDD-Beleuchtungsfilter mit einem AF-Beobachtungsfilter oder ein AF-Beleuchtungsfilter mit einem PDD-Beobachtungsfilter kombiniert ist. Im ersten Fall wird ein extrem dunkles Bild beobachtet, im zweiten Fall ein im Vergleich zu korrekten Kombinationen von Beleuchtungsfilter und Beobachtungsfilter zu helles Bild. Kaum unterscheidbar hingegen ist, ob ein Beleuchtungsfilter für PDD mit einem Beobachtungsfilter für PDD oder ein Beleuchtungsfilter für AF mit einem Beobachtungsfilter für AF kombiniert ist. In beiden Fällen ist das Bild näherungsweise gleich hell, der Unterschied in der Wellenlänge ist für das menschliche Auge allenfalls unter sehr guten Bedingungen im unmittelbaren Vergleich unterscheidbar.

Figur 6 zeigt eine schematische Darstellung eines Reflexionsspektrums 95 des Indikatorbereichs 75 und eines Reflexionsspektrums 96 des Referenzbereichs 76 an der Referenzoberfläche 72. Der Abszisse ist die Wellenlänge λ zugeordnet, der Ordinate der Reflexionsgrad in beliebigen Einheiten. Außerdem sind das Produkt 87 aus dem Transmissionsspektrum 83L des Beleuchtungsfilters für PDD und dem Transmissionsspektrum 84L des Beobachtungsfilters für PDD und das Produkt 88 aus dem Transmissionsspektrum 83F des Beleuchtungsfilters für AF und dem Transmissionsspektrum 84F des Beobachtungsfilters für AF dargestellt. Die Reflexionsspektren 95, 96 weisen jeweils eine Flanke bzw. eine Kante bei 440 nm bis 450 nm auf. Der Reflexionsgrad 95 des Indikatorbereichs 75 weist bei Wellenlängen kleiner als 440 nm einen hohen Wert und bei Wellenlängen größer als 450 nm einen kleinen Wert auf. Der Reflexionsgrad 96 des Referenzbereichs 76 weist bei Wellenlängen kleiner als 440 nm einen kleinen Wert und bei Wellenlängen größer als 450 nm einen großen Wert auf.

Wenn die Referenzoberfläche 72 mit dem Indikatorbereich 75 und dem Referenzbereich 76 mit dem Beleuchtungsfilter 24 für PDD beleuchtet wird, erscheint durch das Beobachtungsfilter für PDD der Indikatorbereich 75 deutlich heller als der Referenzbereich 76. Wenn die Referenzoberfläche 72 durch das Beleuchtungsfilter für AF beleuchtet wird, erscheint durch das Beobachtungsfilter für AF der Indikatorbereich 75 deutlich dunkler als der Referenzbereich 76. Auf diesen unterschiedlichen Helligkeiten beruht das nachfolgend beschriebene Prüfverfahren.

Das distale Ende 12 einer bildgebenden Einrichtung 10 wird in einen Hohlraum 42 in einem Gehäuse 41 einer Prüfvorrichtung 40 eingeführt. Eine Positionierungseinrichtung 50 hält die bildgebende Einrichtung 10, insbesondere deren distales Ende 12, kraft- und/oder formschlüssig an einer vorbestimmten Position und in einer vorbestimmten Richtung relativ zu der beschriebenen Referenzoberfläche 72 mit dem Indikatorbereich 75 und dem Referenzbereich 76, die anhand der Figur 6 beschrieben wurden.

Die Referenzoberfläche wird dann von einer Lichtquelle 22 einer Lichtquellenvorrichtung 20 mit Beleuchtungslicht beleuchtet und durch die bildgebende Einrichtung 10 visuell oder mittels einer Kamera beobachtet. Anhand des dabei erfassten Bilds der Referenzoberfläche kann bestimmt werden, mit welchem Beleuchtungsspektrum die Referenzoberfläche beleuchtet wird bzw. welches Beleuchtungsfilter im Beleuchtungs-Strahlengang vorliegt, und welches Transmissionsspektrum im Beobachtungs-Strahlengang vorliegt. Wenn im erfassten Bild sowohl der Indikatorbereich 75 als auch der Referenzbereich 76 dunkel erscheinen, liegen ein Beleuchtungsspektrum für PDD und ein Beobachtungsfilter für AF vor. Wenn der Indikatorbereich 75 deutlich heller erscheint als der Referenzbereich 76, liegen ein Beleuchtungsspektrum für PDD und ein Beobachtungsfilter für PDD vor. Wenn der Referenzbereich 76 deutlich heller erscheint als der Indikatorbereich 75, liegen ein Beleuchtungsspektrum für AF und ein Beobachtungsfilter für AF vor. Wenn sowohl der Indikatorbereich 75 als auch der Referenzbereich 76 hell erscheinen, liegen ein Beleuchtungsspektrum für AF und ein Beobachtungsfilter für PDD vor.

Die beschriebene Referenzoberfläche 72 mit dem Indikatorbereich 75 und dem Referenzbereich 76 kann vielfältig variiert werden. Beispielsweise wenn lediglich eine Unterscheidung zwischen den beiden zulässigen Filterkombinationen (PDD-Beleuchtungsfilter und PDD-Beobachtungsfilter oder AF-Beleuchtungsfilter und AF-Beobachtungsfilter) gewünscht ist, kann auf den Referenzbereich 76 verzichtet werden. Der Indikatorbereich 75 kann sogar die gesamte Fläche der Referenzoberfläche 72 einnehmen. Allein anhand einer Erkennung, ob der Indikatorbereich 75 im erfassten Bild hell oder dunkel erscheint, kann unterschieden werden, welche der beiden zulässigen Filterkombinationen vorliegt. Die unzulässigen Filterkombinationen können dann beispielsweise anhand anderer Merkmale der Referenzoberfläche 72 identifiziert werden.

Alternativ zu einem Helligkeitskontrast oder zusätzlich zu diesem kann ein Farbkontrast erzeugt werden. Beispielsweise weist der Indikatorbereich 75 ein Transmissionsfilter vor einer fluoreszierenden Fläche auf, wobei der Transmissionsgrad des Indikatorfilters wie der Reflexionsgrad 76 aus dem oben dargestellten Beispiel eine ansteigende Flanke im Bereich von 440 nm bis 450 nm aufweist. Der Reflexionsgrad R (λ) beträgt näherungsweise R (λ) = 1-T (λ) und hat damit näherungsweise die Gestalt des Reflexionsspektrums 95 aus dem oben beschriebenen Beispiel. Die fluoreszierende Fläche unter bzw. hinter dem Indikatorfilter zeige eine Fluoreszenz, die durch Wellenlängen kleiner 440 nm angeregt werden kann, wobei das Fluoreszenzlicht beispielsweise im grünen oder roten Spektralbereich liegt. Bei Beleuchtung dieses Indikatorbereichs mit einem PDD-Beleuchtungsspektrum wird dieses ohne Frequenzverschiebung reflektiert. Bei Beleuchtung des Indikatorbereichs mit AF-Beleuchtungslicht wird ein Teil des Beleuchtungslichts (dessen langwelliger Anteil) durch das Indikatorfilter auf die darunterliegende fluoreszierende Fläche fallen und diese zur Fluoreszenz anregen. Das Fluoreszenzlicht kann das Indikatorfilter passieren. Somit erscheint der Indikatorbereich 75 im erfassten Bild bei Beleuchtung mit einem PDD-Beleuchtungsspektrum und Beobachtung durch ein PDD-Beobachtungsfilter blau und bei Beleuchtung mit einem AF-Beleuchtungsspektrum und Beobachtung durch ein AF-Beobachtungsspektrum grün bis rot.

Wenn eine Kamera 31 verwendet wird, die dies zulässt, kann vor dem Erfassen des Bilds die Belichtungszeit oder die Verstärkung fest vorgegeben werden und lediglich der sich dabei einstellende jeweils andere Parameter erfasst und ausgewertet werden. Dadurch können beispielsweise eine Kombination eines PDD-Beleuchtungsspektrums mit einem AF-Beobachtungsfilter (vgl. Kurve 85 in Figur 5) und eine Kombination eines AF-Beleuchtungsspektrums mit einem PDD-Beobachtungsfilter (vgl. Kurve 86 in Figur 5) durch eine zumindest semiquantitative Auswertung der Helligkeit eines weißen Bereichs der Referenzoberfläche 72 im erfassten Bild unterschieden bzw. identifiziert werden.

Im Rahmen des beschriebenen Prüfverfahrens können, insbesondere über eine Benutzerschnittstelle, Patientendaten erfasst und anschließend gemeinsam mit dem Ergebnis des Prüfverfahrens und insbesondere zusammen mit dem Ergebnis einer nachfolgenden Untersuchung des Patienten mittels des optischen Untersuchungssystems in einer Datenbank abgelegt werden. Damit wird sichergestellt, dass das optische Untersuchungssystem vor oder nach der Untersuchung eines Patienten auf seine Funktionsfähigkeit geprüft und das Ergebnis dieser Prüfung protokolliert bzw. dokumentiert wird.

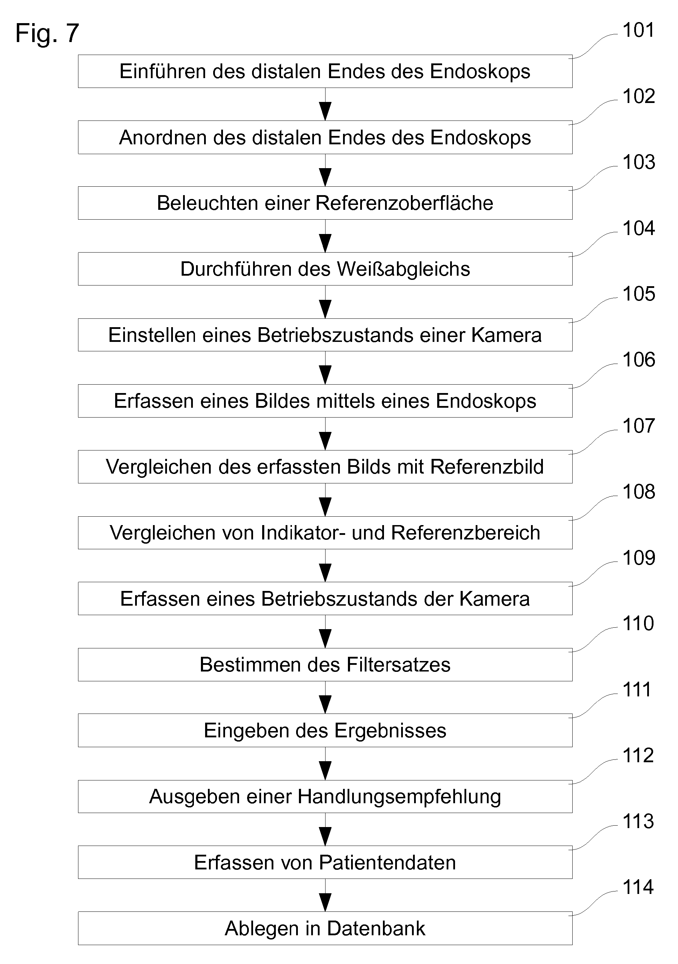

Figur 7 zeigt ein schematisches Flussdiagramm eines Verfahrens zum Prüfen eines optischen Untersuchungssystems mit einer Lichtquelle, einer bildgebenden Einrichtung und einer Kamera zur optischen Untersuchung eines Objekts. Obwohl das Verfahren auch an optischen Untersuchungssystemen und Prüfvorrichtungen anwendbar ist, die sich von dem oben anhand der Figuren 1 und 2 dargestellten unterscheiden, werden nachfolgend zur Vereinfachung des Verständnisses beispielhaft Bezugszeichen aus den Figuren 1 und 2 verwendet. Das Verfahren kann Merkmale der oben beschriebenen Prüfverfahren und seiner beschriebenen Varianten aufweisen. Insbesondere kann das Verfahren eine Kombination mehrerer beschriebener Varianten sein.

Bei einem optionalen ersten Schritt 101 wird ein distales Ende 12 einer bildgebenden Einrichtung 10, insbesondere eines Endoskops, durch eine Öffnung 43 in einen Hohlraum 42 in einem lichtdichten Gehäuse 41 eingeführt. Bei einem optionalen zweiten Schritt 102, der unmittelbar nach dem ersten Schritt 101 oder gleichzeitig mit diesem ausgeführt werden kann, wird das distale Ende 12 der bildgebenden Einrichtung 10 in einer vorbestimmten Position und Richtung relativ zu einer in dem Hohlraum 42 angeordneten Referenzoberfläche 72 angeordnet. Dies erfolgt beispielsweise unterstützt durch eine Positionierungseinrichtung 50, die die bildgebende Einrichtung 10, insbesondere deren distales Ende 12 führt und/oder form- oder kraftschlüssig hält.

Alternativ werden die nachfolgenden Schritte ausgeführt ohne das distale Ende 12 der bildgebenden Einrichtung 10 zuvor in einen Hohlraum 42 einzuführen und/oder ohne das distale Ende 12 der bildgebenden Einrichtung 10 zuvor genau zu positionieren.

Bei einem dritten Schritt 103 wird die Referenzoberfläche 72 mit Beleuchtungslicht mit einem Beleuchtungsspektrum beleuchtet. Wenn die bildgebende Einrichtung ein Endoskop 10 ist, erfolgt die Beleuchtung insbesondere mittels des Endoskops 10 bzw. über einen Beleuchtungs-Strahlengang im Endoskop 10.

Wenn eine Kamera 31 verwendet wird, kann bei einem optionalen vierten Schritt 104 ein Weißabgleich durchgeführt werden während die Referenzoberfläche beleuchtet wird. Dabei werden beispielsweise Weißabgleich-Parameter WBGr, WBGb eingestellt. Wenn eine Kamera 31 verwendet wird, kann bei einem optionalen fünften Schritt 105 ein Betriebszustand der Kamera 31 eingestellt werden. Beispielsweise werden die Belichtungszeit, eine Verstärkung oder andere die Belichtung, die optisch-elektrische Wandlung und die Digitalisierung von Bildern betreffende Parameter auf vorbestimmte Werte eingestellt.

Bei einem sechsten Schritt 106 wird während des Beleuchtens der Referenzoberfläche 72 mittels der bildgebenden Einrichtung 10 durch eine Kamera 31 oder unmittelbar visuell bzw. durch das menschliche Auge ein Bild erfasst. Im zweiten Fall entfällt unter anderem der fünfte Schritt 105.

Bei einem optionalen siebten Schritt 107 wird das beim sechsten Schritt 106 mittels der bildgehenden Einrichtung 10 erfasste Bild mit einem Referenzbild, insbesondere mit mehreren Referenzbildern, verglichen. Bei einem ebenfalls optionalen achten Schritt 108 wird innerhalb des beim sechsten Schritt 106 erfassten Bild die Erscheinung eines Indikatorbereichs mit der Erscheinung eines Referenzbereichs verglichen.

Wenn eine Kamera 31 verwendet wird, kann bei einem optionalen neunten Schritt 109 ein während des sechsten Schritts 106 vorliegender Betriebszustand der Kamera erfasst werden. Der beim Erfassen des Bilds vorliegende Betriebszustand ermöglicht für sich allein oder zusammen mit dem erfassten Bild beispielsweise eine zumindest semiquantitative Aussage über die Helligkeit des von der bildgebenden Einrichtung 10 in der Kamera erzeugten Bilds oder eines Bereichs innerhalb dieses Bilds. Beispielsweise kann auf der Grundlage der Belichtungszeit, der Verstärkung und der Helligkeitswerte im erfassten Bild bei bekannten Eigenschaften der Referenzoberfläche 72 und bekannter Positionierung des distalen Endes 12 der bildgebenden Einrichtung relativ zur Referenzoberfläche 72 eine zumindest semiquantitative Aussage abgeleitet werden. Beispielsweise kann auf dieser Grundlage bei dem anhand der Figur 5 dargestellten Beispiel unterschieden werden, ob eine zulässige Filterkombination oder eine unzulässige Filterkombination vorliegt.

Bei einem zehnten Schritt 110 wird bestimmt, welches Beleuchtungsspektrum und welches Transmissionsspektrum im Beobachtungs-Strahlengang in dem optischen Untersuchungssystem vorliegen, insbesondere welches Beleuchtungsfilter und welches Beobachtungsfilter vorliegen. Diese Aussage kann insbesondere gewonnen werden aus der Erscheinung des Indikatorbereichs im erfassten Bild, aus dem Ergebnis des Vergleichs des erfassten Bilds mit einem Referenzbild (siebter Schritt 107) und/oder aus dem Ergebnis des Vergleichs der Erscheinungen des Indikatorbereichs und des Referenzbereichs im erfassten Bild (achter Schritt 108). Wenn das Bild mit einer Kamera erfasst wurde, können in die Bestimmung des Beleuchtungsspektrums und des Transmissionsspektrums im Beobachtungs-Strahlengang alternativ oder zusätzlich einfließen die beim vierten Schritt 104 ermittelten Weißabgleich-Parameter, der beim fünften Schritt 105 eingestellte Betriebszustand der Kamera 31 und/oder der beim neunten Schritt 109 erfasste Betriebszustand der Kamera 31.

Wenn beim sechsten Schritt 106 das Bild unmittelbar visuell erfasst wurde, können bei einem optionalen elften Schritt über eine Benutzerschnittstelle Eigenschaften des erfassten Bilds oder das Ergebnis eines Vergleichs des erfassten Bilds mit einem oder mehreren Referenzbildern abgefragt und erfasst und in eine Datenverarbeitung übernommen werden. Wenn beim sechsten Schritt 106 das Bild durch eine Kamera erfasst wurde, kann der zehnte Schritt durch eine Vorrichtung, insbesondere durch eine Kamerasteuerung 35 oder durch einen Computer, ausgeführt werden. In diesem Fall kann das Ergebnis des zehnten Schritts bereits in einer Form vorliegen, die die nachfolgenden Schritte ermöglicht.

Bei einem optionalen zwölften Schritt 112 wird eine Meldung ausgegeben, die eine Aussage über die Funktionsfähigkeit (insbesondere zulässige und richtige Kombination von Beleuchtungsfilter und Beobachtungsfilter), über die verwendeten Filter bzw. über das Beleuchtungsspektrum und das Transmissionsspektrum im Beobachtungs-Strahlengang umfassen kann. Ferner kann die Meldung eine Handlungsempfehlung und/oder eine Handlungsanweisung umfassen. Beispielsweise umfasst die Meldung eine Aufforderung, das Beleuchtungsfilter gegen ein anderes Beleuchtungsfilter oder die bildgebende Einrichtung gegen eine andere bildgebende Einrichtung mit einem anderen Transmissionsspektrum auszutauschen.

Bei einem optionalen dreizehnten Schritt 113, der auch zu einem beliebigen anderen Zeitpunkt in dem Verfahren ausgeführt werden kann, werden Patientendaten erfasst, beispielsweise mittels einer Benutzerschnittstelle. Bei einem optionalen vierzehnten Schritt 114 werden die Patientendaten, das Ergebnis des Prüfverfahrens und optional das Ergebnis einer nachfolgenden oder vorangehenden Untersuchung eines Patienten mittels des optischen Untersuchungssystems in einer Datenbank abgelegt.

Insbesondere bei Verwendung einer Kamera 31 können ferner Modellbezeichnungen, Seriennummern, Software- oder Firmware-Versionen und andere Daten von Komponenten des optischen Untersuchungssystems über eine Kommunikationsleitung 39 abgefragt und zur Dokumentation bzw. Protokollierung in der Datenbank abgelegt werden. Ferner kann in der Datenbank oder separat auf einem anderen Datenträger die Untersuchung des Patienten dokumentiert bzw. protokolliert werden. Dabei werden beispielsweise Bilder bzw. ein Videodatenstrom der Kamera 31 in der Datenbank (beispielsweise in einem MPEG-Format) oder auf einem Videoband abgelegt.

### Bezugszeichen

- 10: Endoskop
- 11: proximales Ende des Endoskops 10
- 12: distales Ende des Endoskops 10
- 13: Beobachtungsfilter des Endoskops 10
- 14: Okular am proximalen Ende des Endoskops 10
- 15: Kupplung am Endoskop 10 für Lichtleitkabel 19
- 17: Schaft des Endoskops 10
- 19: Lichtleitkabel zu Kopplung des Endoskops 10 mit der Lichtquellenvorrichtung 20
- 20: Lichtquellenvorrichtung
- 22: Lichtquelle der Lichtquellenvorrichtung 20
- 23: erste Sammellinse
- 24: Beleuchtungsfilter der Lichtquellenvorrichtung 20
- 25: zweite Sammellinse
- 26: Kupplung an der Lichtquellenvorrichtung 20 für Lichtleitkabel 19

- 31: Kamera
- 33: Signalkabel
- 35: Kamerasteuerung
- 37: Bildschirm
- 39: Kommunikationsleitung
- 40: Prüfvorrichtung
- 41: lichtdichtes Gehäuse der Prüfvorrichtung 40
- 42: Hohlraum im lichtdichten Gehäuse 41
- 43: Öffnung im lichtdichten Gehäuse 41

- 50: erste Positionierungseinrichtung

- 70: Referenzkörper der Prüfvorrichtung 40
- 72: Referenzoberfläche am Referenzkörper 70
- 74: Stift am Referenzkörper 70
- 75: Indikatorbereich an der Referenzfläche 72
- 76: Referenzbereich an der Referenzfläche 72

- 81: Fluoreszenz-Anregungsspektrum
- 82: Fluoreszenz-Abregungsspektrum
- 83: Transmissionsspektrum des Beleuchtungsfilters
- 83L: Transmissionsspektrum des Beleuchtungsfilters für PDD

- 83F: Transmissionsspektrum des Beleuchtungsfilters für AF
- 84: Transmissionsspektrum des Beobachtungsfilters
- 84L: Transmissionsspektrum des Beobachtungsfilters für PDD
- 84F: Transmissionsspektrum des Beobachtungsfilters für AF
- 85: Produkt aus 83L und 84F
- 86: Produkt aus 83F und 84L
- 87: Produkt aus 83L und 84L
- 88: Produkt aus 83F und 84F

- 93: Reflexionsspektrum des Indikatorbereichs 73
- 95: Reflexionsspektrum des Referenzbereichs 75

- 101: erster Schritt (Einführen des distalen Endes des Endoskops)
- 102: zweiter Schritt (Anordnen des distalen Endes des Endoskops)
- 103: dritter Schritt (Beleuchten einer Referenzoberfläche)
- 104: vierter Schritt (Durchführen eines Weißabgleichs)
- 105: fünfter Schritt (Einstellen eines Betriebszustands einer Kamera)
- 106: sechster Schritt (Erfassen eines Bilds mittels Endoskop und Kamera)
- 107: siebter Schritt (Vergleichen des erfassten Bilds mit Referenzbild)
- 108: achter Schritt (Vergleichen von Indikator- und Referenzbereich)
- 109: neunter Schritt (Erfassen eines Betriebszustands der Kamera)
- 110: zehnter Schritt (Bestimmen des Filtersatzes)
- 111: elfter Schritt (Eingeben eines Ergebnisses)
- 112: zwölfter Schritt (Ausgeben einer Handlungsempfehlung)
- 113: dreizehnter Schritt (Erfassen von Patientendaten)
- 114: vierzehnter Schritt (Ablegen in Datenbank)

## Patentansprüche

1. **Verfahren zum Prüfen** eines optischen Untersuchungssystems mit einer Lichtquelle (22) und einer bildgebenden Einrichtung (10) zur optischen Untersuchung eines Objekts in remittiertem Licht und Fluoreszenzlicht, wobei
die Lichtquelle (22) ausgebildet ist, um Beleuchtungslicht zumindest entweder mit einem ersten vorbestimmten Beleuchtungsspektrum (83L) oder mit einem zweiten vorbestimmten Beleuchtungsspektrum (83F) zu erzeugen,
ein Beobachtungs-Strahlengang der bildgebende Einrichtung (10) zumindest entweder ein erstes vorbestimmtes Transmissionsspektrum (84L) oder ein zweites vorbestimmtes Transmissionsspektrum (84F) aufweist,
mit folgenden Schritten:
**Anordnen** (102) der bildgebenden Einrichtung gegenüber einer Referenzoberfläche (72) mit einem **Indikatorbereich** (75) mit einer wellenlängenabhängigen optischen Eigenschaft, die sich zwischen einem ersten Schwerpunkt eines ersten Produkts (87) aus dem ersten vorbestimmten Beleuchtungsspektrum (83L) und dem ersten vorbestimmten Transmissionsspektrum (84L) und einem zweiten Schwerpunkt eines zweiten Produkts (88) aus dem zweiten vorbestimmten Beleuchtungsspektrums (83F) und dem zweiten vorbestimmten Transmissionsspektrum (84F) wesentlich ändert;
**Beleuchten** (103) der Referenzoberfläche (72) mit Beleuchtungslicht der Lichtquelle (22);
Erfassen (106) eines **Bilds** der Referenzoberfläche (72) mittels der bildgebenden Einrichtung (10);
Bestimmen (110), welches Beleuchtungsspektrum und welches Transmissionsspektrum im Beobachtungs-Strahlengang beim Erfassen (106) des Bilds vorlag, anhand des erfassten Bilds.

2. Verfahren nach dem vorangehenden Anspruch, bei dem der Schritt des Bestimmens (110) ein **Vergleichen** des erfassten Bilds mit einem Referenzbild umfasst.

3. Verfahren nach einem der vorangehenden Ansprüche, bei dem die bildgebende Einrichtung (10) gegenüber einer Referenzoberfläche (72) angeordnet wird, die neben dem Indikatorbereich (75) einen Referenzbereich (76) aufweist, wobei der Schritt des Bestimmens (110) ein **Vergleichen** einer Wiedergabe des Indikatorbereichs (75) mit einer Wiedergabe des Referenzbereichs (76) im erfassten Bild umfasst.

4. Verfahren nach einem der vorangehenden Ansprüche, bei dem eine optische Eigenschaft des Indikatorbereichs (75) und eine optische Eigenschaft eines Referenzbereichs (76) an der Referenzoberfläche (72) sich zwischen dem ersten Schwerpunkt und dem zweiten Schwerpunkt gegenläufig ändern.

5. Verfahren nach dem vorangehenden Anspruch, bei dem der Indikatorbereich (75) ein Indikatorfilter umfasst, dessen **Filterkante zwischen** dem ersten Schwerpunkt und dem zweiten Schwerpunkt liegt.

6. Verfahren nach dem vorangehenden Anspruch, bei dem der Indikatorbereich (75) eine **fluoreszierende Fläche** hinter dem Indikatorfilter umfasst.

7. Verfahren nach einem der vorangehenden Ansprüche, bei dem das Bild der Referenzoberfläche (72) visuell oder durch eine **Kamera** (31) erfasst wird.

8. Verfahren nach einem der vorangehenden Ansprüche, ferner mit folgendem Schritt:
**Einstellen (105) eines Betriebszustands** einer Kamera (31) auf einen vorbestimmten Wert vor dem Erfassen (106) des Bilds der Referenzoberfläche (72) durch die Kamera (31).

9. Verfahren nach einem der vorangehenden Ansprüche, ferner mit folgendem Schritt:
**Erfassen** (109) eines während des Erfassens des Bilds durch eine Kamera (31) vorliegenden **Betriebszustands** der Kamera (31).

10. Verfahren nach einem der vorangehenden Ansprüche, ferner mit folgenden Schritten:
Erfassen (113) von Patientendaten;
Ablegen (114) einer Information über das Beleuchtungsspektrum (83) und das Transmissionsspektrum (84) im Beobachtungs-Strahlengang sowie der Patientendaten in einer Datenbank.

11. **Referenzkörper** (70) zum Prüfen eines optischen Untersuchungssystems mit einer Lichtquelle (22) zum Erzeugen eines ersten vorbestimmten Beleuchtungsspektrums (83L) oder eines zweiten vorbestimmten Beleuchtungsspektrums (83F) und einer bildgebenden Einrichtung (10) mit einem Beobachtungs-Strahlengang mit einem ersten vorbestimmten Transmissionsspektrum (84L) oder einem zweiten vorbestimmten Transmissionsspektrum (84F), mit:
einer Referenzoberfläche (72);
einem Indikatorbereich (75) an der Referenzoberfläche (72), wobei eine wellenlängenabhängige optische Eigenschaft des Indikatorbereichs (75) sich zwischen einem ersten Schwerpunkt eines ersten Produkts (87) aus dem ersten vorbestimmten Beleuchtungsspektrum (83L) und dem ersten vorbestimmten Transmissionsspektrum (84L) und einem zweiten Schwerpunkt eines zweiten Produkts (88) aus dem zweiten vorbestimmten Beleuchtungsspektrum (83F) und dem zweiten vorbestimmten Transmissionsspektrum (84F) wesentlich ändert.

12. Referenzkörper (70) nach dem vorangehenden Anspruch, wobei der Referenzkörper (70) zur Durchführung eines Verfahrens nach einem der Ansprüche 1 bis 10 ausgebildet ist.
